# EUROPEAN PATENT APPLICATION

(11) **EP 3 711 643 A1**
(43) Date of publication of application: **23.09.2020**
(21) Application number: 19020170.7
(22) Date of filing: 22.03.2019
(51) Int. Cl.: A47J 31/44, A24F 47/00, A61M 11/04, A61M 15/00, A61M 15/06

(54) **SMOKING SUBSTITUTE SYSTEM**

(71) Applicant: NERUDIA LIMITED, Liverpool Merseyside L24 9HP (GB)
(72) Inventor: FERRIE, Kate, Speke, Liverpool L24 9HP (GB); SHENTON, Ross, Speke, Liverpool L24 9HP (GB)
(74) Representative: Mewburn Ellis LLP

(57) **Abstract**

The present disclosure relates to a smoking substitute device for receiving a heated tobacco consumable. The device is configured to receive a first user input to define one or more parameters (e.g. amplitude, frequency, duration or pattern/sequence) of a first haptic feedback output; and output the first haptic feedback output at or proximal the conclusion of the consumable smoking cycle.

## Description

### TECHNICAL FIELD

The present invention relates to a smoking substitute and a method of controlling the operation of the smoking substitute device.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles (also referred to as a "consumables") that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories.

There are a number of different categories of smoking substitute systems, each utilising a different smoking substitute approach.

One approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

There may be a need for improved design of smoking substitute systems, in particular HT smoking substitute systems, to enhance the user experience and improve the function of the HT smoking substitute system.

The present disclosure has been devised in the light of the above considerations.

### SUMMARY OF THE INVENTION

At its most general, the present invention relates to operating a smoking substitute device to provide a user-defined haptic feedback output during a consumable smoking routine.

According to a first aspect of the present invention, there is provided a smoking substitute device for receiving a heated tobacco consumable, the device configured to receive a first user input to define one or more parameters of a first haptic feedback output; and output the first haptic feedback output at or proximal the conclusion of the consumable smoking cycle.

The device provides a first haptic feedback output (e.g. vibration) at or near the end of the consumable smoking cycle to alert the user that the consumable is spent. By allowing the user to define the parameter(s) of the first haptic feedback (with the first user input), the user experience is enhanced by the ability to define a haptic feedback of choice.

Optional features will now be set out. These are applicable singly or in any combination with any aspect.

In some embodiments, the device comprises user input means for receiving the first user input. The first user input may define the amplitude and/or the frequency and/or the duration and/or the pulse pattern/sequence of the first haptic feedback output. The input means may be configured to generate a first user input signal upon receipt of the first user input.

The input means may be provided on a user interface (UI). In some embodiments the UI may comprise a power button to switch the device between an ON state and an OFF state.

The device may comprise a controller for receiving the first user input signal from the user input means. The controller may comprise a microcontroller that may e.g. be mounted on a printed circuit board (PCB). The controller may also comprise a memory, e.g. non-volatile memory for storing the first haptic feedback output parameters defined in the first user input.

Conveniently, the device further comprises feedback means having a haptic feedback element for generating the first haptic feedback output upon receipt of a 'spent' output signal from the controller. The feedback means may be provided on the Ul. The controller is configured to generate the spent output signal after a time period determined by the time since the commencement of the consumable smoking cycle and/or the number of puffs taken on the consumable by the user and/or the flow rate/duration of draw in each puff.

In some embodiments, the device comprises a puff sensor (e.g. airflow sensor) for detecting if the user has puffed on the consumable. The puff sensor may be operatively connected to the controller and may send a puff signal to the controller upon each puff by the user. The controller may be configured to control a heater within the device based on the puff signal from the puff sensor.

The puff sensor may, for example, be a pressure sensor or a microphone. The puff signal may be indicative of the user drawing (an aerosol from the consumable) such that it is in the form of a binary signal. Alternatively or additionally, the signal may be indicative of a characteristic of the draw (e.g. a flow rate of the draw, length of time of the draw, etc.).

In some embodiments, the device is further configured to output a second haptic feedback output if the user has not puffed on the consumable for a predetermined length of time during the consumable smoking cycle (i.e. if the time elapsed between puff signals exceeds the predetermined length of time). The predetermined length of time may be greater than 30 seconds, e.g. 1 minute or greater.

The controller may comprise a timer to detect the time elapsed between puff signals from the puff sensor. Where the elapsed time reaches the predetermined length of time, the controller may send an 'idle' output signal to the haptic element in the feedback means to generate the second haptic feedback output.

The device may be configured to receive a second user input (e.g. at the user input means) to define one or more parameters of the second haptic feedback output; and output the second haptic feedback output when the length of time between consecutive puffs by the user on the consumable exceeds the predetermined time.

In preferred embodiments, the second haptic feedback output has different parameters (e.g. amplitude, frequency, duration, pulse pattern/sequence) than the first haptic feedback output. The defined parameter(s) for the second haptic feedback may be stored in the memory of the controller.

In some embodiments, the device comprises a power source or may be connectable to a power source (e.g. a power source separate to the device) and the device is further configured to output a third haptic feedback output when the power source is depleted or is approaching depletion.

The controller may send a 'depletion' output signal to the haptic element in the feedback means to generate the third haptic feedback output using the haptic element in the feedback means.

The device may be configured to receive a third user input (e.g. at the user input means) to define one or more parameters of the third haptic feedback output; and output the third haptic feedback output when the power supply (e.g. battery) is approaching depletion.

In preferred embodiments, the third haptic feedback output has different parameters (e.g. amplitude, frequency, duration, pulse pattern/sequence) than the first and/or second haptic feedback output. The defined parameter(s) for the third haptic feedback may be stored in the memory of the controller.

The power source may be a power store. For example, the power source may be a battery or rechargeable battery (e.g. a lithium ion battery).

The device may comprise an input connection (e.g. a USB port, Micro USB port, USB-C port, etc.). The input connection may be configured for connection to an external source of electrical power, such as a mains electrical supply outlet. The input connection may, in some cases, be used as a substitute for an internal power source (e.g. battery or rechargeable battery). That is, the input connection may be electrically connectable to a heater (for providing power to the heater). Hence, in some forms, the input connection may form at least part of the power source of the device.

Where the power source comprises a rechargeable power source (such as a rechargeable battery), the input connection may be used to charge and recharge the power source.

In some embodiments, the feedback means further comprises an audio feedback element and/or a visual feedback element. For example, the visual feedback element may comprise one or more (e.g. two, three, four, etc.) light-emitting diodes ("LEDs") that may be located on the body of the device (e.g. on the Ul). One or both of the visual/audio feedback elements can be configured to provide a first, second or third visual/audio feedback output simultaneously to the first, second or third haptic feedback output respectively.

Accordingly, the first user input may define one or more parameters of the first visual and/or first audio feedback output; and the device may be configured to output the first visual and/or audio feedback output simultaneously with the first haptic feedback output.

Similarly, the second/third user input may define one or more parameters of the second/third visual and/or second/third audio feedback output; and the device may be configured to output the second/third visual and/or audio feedback output simultaneously with the second/third haptic feedback output respectively.

In preferred embodiments, the first, second and third audio and/or visual feedback outputs each has different parameters (e.g. amplitude, frequency, duration, pulse pattern/sequence) than each other.

The device may comprise an elongate body. An end of the elongate body may be configured for engagement with the heated tobacco (HT)/heat not burn (HNB) consumable. The device may comprise a cavity that is configured for receipt of at least a portion of the consumable (i.e. for engagement with the consumable). The consumable may be of the type that comprises an aerosol former (e.g. carried by an aerosol-forming substrate).

The device may comprise a heater for heating the consumable. The heater may comprise a heating element, which may be in the form of a rod that extends from the body of the device. The heating element may extend from the end of the body that is configured for engagement with the consumable.

The heater (and thus the heating element) may be rigidly mounted to the body. The heating element may be elongate so as to define a longitudinal axis and may, for example, have a transverse profile (i.e. transverse to a longitudinal axis of the heating element) that is substantially circular (i.e. the heating element may be generally cylindrical). Alternatively, the heating element may have a transverse profile that is rectangular (i.e. the heater may be a "blade heater"). The heating element may alternatively be in the shape of a tube (i.e. the heater may be a "tube heater"). The heating element may take other forms (e.g. the heating element may have an elliptical transverse profile). The shape and/or size (e.g. diameter) of the transverse profile of the heating element may be generally consistent for the entire length (or substantially the entire length) of the heating element.

The heating element may be between 15 mm and 25 mm long, e.g. between 18 mm and 20 mm long, e.g. around 19 mm long. The heating element may have a diameter of between 1.5 mm and 2.5 mm, e.g. a diameter between 2 mm and 2.3 mm, e.g. a diameter of around 2.15 mm.

The heating element may be formed of ceramic. The heating element may comprise a core (e.g. a ceramic core) comprising Al2O3. The core of the heating element may have a diameter of 1.8 mm to 2.1 mm, e.g. between 1.9 mm and 2 mm. The heating element may comprise an outer layer (e.g. an outer ceramic layer) comprising Al2O3. The thickness of the outer layer may be between 160 µm and 220 µm, e.g. between 170 µm and 190 µm, e.g. around 180 µm. The heating element may comprise a heating track, which may extend longitudinally along the heating element. The heating track may be sandwiched between the outer layer and the core of the heating element. The heating track may comprise tungsten and/or rhenium. The heating track may have a thickness of around 20 µm.

The heating element may be located in the cavity (of the device), and may extend (e.g. along a longitudinal axis) from an internal base of the cavity towards an opening of the cavity. The length of the heating element (i.e. along the longitudinal axis of the heater) may be less than the depth of the cavity. Hence, the heating element may extend for only a portion of the length of the cavity. That is, the heating element may not extend through (or beyond) the opening of the cavity.

The heating element may be configured for insertion into the consumable when it is received in the cavity. In that respect, a distal end (i.e. distal from a base of the heating element where it is mounted to the device) of the heating element may comprise a tapered portion, which may facilitate insertion of the heating element into the consumable. The heating element may fully penetrate the consumable when it is received in the cavity. That is, the entire length, or substantially the entire length, of the heating element may be received in the consumable.

The heating element may have a length that is less than, or substantially the same as, an axial length of an aerosol-forming substrate forming part of the consumable. Thus, when such a consumable is engaged with the device, the heating element may only penetrate the aerosol-forming substrate, rather than other components of the consumable. The heating element may penetrate the aerosol-forming substrate for substantially the entire axial length of the aerosol forming-substrate of the consumable. Thus, heat may be transferred from (e.g. an outer circumferential surface of) the heating element to the surrounding aerosol-forming substrate, when penetrated by the heating element. That is, heat may be transferred radially outwardly (in the case of a cylindrical heating element) or e.g. radially inwardly (in the case of a tube heater).

Where the heater is a tube heater, the heating element of the tube heater may surround at least a portion of the cavity. When the portion of the consumable is received in the cavity, the heating element may surround a portion of the consumable (i.e. so as to heat that portion of the consumable). In particular, the heating element may surround an aerosol forming substrate of the consumable. That is, when the consumable is engaged with the device, the aerosol forming substrate of the consumable may be located adjacent an inner surface of the (tubular) heating element. When the heating element is activated, heat may be transferred radially inwardly from the inner surface of the heating element to heat the aerosol forming substrate.

The cavity may comprise a (e.g. circumferential) wall (or walls) and the (tubular) heating element may extend around at least a portion of the wall(s). In this way, the wall may be located between the inner surface of the heating element and an outer surface of the consumable. The wall (or walls) of the cavity may be formed from a thermally conductive material (e.g. a metal) to allow heat conduction from the heating element to the consumable. Thus, heat may be conducted from the heating element, through the cavity wall (or walls), to the aerosol-forming substrate of the consumable received in the cavity.

In some embodiments the device may comprise a cap disposed at the end of the body that is configured for engagement with the consumable. Where the device comprises a heater having a heating element, the cap may at least partially enclose the heating element. The cap may be moveable between an open position in which access is provided to the heating element, and a closed position in which the cap at least partially encloses the heating element. The cap may be slideably engaged with the body of the device, and may be slideable between the open and closed positions.

The cap may define at least a portion of the cavity of the device. That is, the cavity may be fully defined by the cap, or each of the cap and body may define a portion of the cavity. The cap may comprise an opening to the cavity. The opening may be configured for receipt of at least a portion of the consumable. That is, the consumable may be inserted through the opening and into the cavity (so as to be engaged with the device).

The cap may be configured such that when the consumable is engaged with the device (e.g. received in the cavity), only a portion of the consumable is received in the cavity. That is, a portion of the consumable (not received in the cavity) may protrude from (i.e. extend beyond) the opening. This (protruding) portion of the consumable may be a terminal (e.g. mouth) end of the consumable, which may be received in a user's mouth for the purpose of inhaling aerosol formed by the device.

The controller may be configured to control the operation of the heater (and e.g. the heating element). Thus, the controller may be configured to control vaporisation of an aerosol forming part of the consumable engaged with the device. The controller may be configured to control the voltage applied by power source to the heater. For example, the controller may be configured to toggle between applying a full output voltage (of the power source) to the heater and applying no voltage to the heater. Alternatively or additionally, the control unit may implement a more complex heater control protocol.

The device may further comprise a voltage regulator to regulate the output voltage supplied by the power source to form a regulated voltage. The regulated voltage may subsequently be applied to the heater.

The device may comprise a wireless interface configured to communicate wirelessly (e.g. via Bluetooth (e.g. a Bluetooth low-energy connection) or Wi-Fi) with an external device. Similarly, the input connection may be configured for wired connection to an external device so as to provide communication between the device and the external device.

The external device may be a mobile device. For example, the external device may be a smart phone, tablet, smart watch, or smart car. An application (e.g. app) may be installed on the external device (e.g. mobile device). The application may facilitate communication between the device and the external device via the wired or wireless connection.

The wireless or wired interface may be configured to transfer signals between the external device and the controller of the device. In this respect, the controller may control an aspect of the device in response to a signal received from an external device. Alternatively or additionally, an external device may respond to a signal received from the device (e.g. from the controller of the device).

In a second aspect, there is provided a system (e.g. a smoking substitute system) comprising a device according to the first aspect and a consumable. The consumable may comprise an aerosol-forming substrate at an upstream end of the consumable.

As used herein, the terms "upstream" and "downstream" are intended to refer to the flow direction of the vapour/aerosol i.e. with the downstream end of the consumable being the mouth end or outlet where the aerosol exits the consumable for inhalation by the user. The upstream end of the consumable is the opposing end to the downstream end.

The aerosol-forming substrate is capable of being heated to release at least one volatile compound that can form an aerosol. The aerosol-forming substrate may be located at the upstream end of the consumable.

In order to generate an aerosol, the aerosol-forming substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The aerosol-forming substrate may comprise plant material. The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus*, *Tumera diffusa* (Damiana), *Verbascum* (Mullein), *Zamia latifolia* (Maconha Brava) together with any combinations, functional equivalents to, and/or synthetic alternatives of the foregoing.

The plant material may be tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise a gathered sheet of homogenised (e.g. paper/slurry recon) tobacco or gathered shreds/strips formed from such a sheet.

The aerosol-forming substrate may comprise one or more additives selected from humectants, flavourants, fillers, aqueous/non-aqueous solvents and binders.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate may be formed in a substantially cylindrical shape such that the article/consumable resembles a conventional cigarette. It may have a diameter of between 5 and 10mm e.g. between 6 and 9mm or 6 and 8mm e.g. around 7 mm. It may have an axial length of between 10 and 15mm e.g. between 11 and 14mm such as around 12 or 13mm.

The article/consumable may comprise at least one filter element. There may be a terminal filter element at the downstream/mouth end of the article/consumable.

The or at least one of the filter element(s) (e.g. the terminal filter element) may be comprised of cellulose acetate or polypropylene tow. The at least one filter element (e.g. the terminal filter element) may be comprised of activated charcoal. The at least one filter element (e.g. the terminal element) may be comprised of paper. The or each filter element may be at least partly (e.g. entirely) circumscribed with a plug wrap e.g. a paper plug wrap.

The terminal filter element (at the downstream end of the article/consumable) may be joined to the upstream elements forming the article/consumable by a circumscribing tipping layer e.g. a tipping paper layer. The tipping paper may have an axial length longer than the axial length of the terminal filter element such that the tipping paper completely circumscribes the terminal filter element plus the wrapping layer surrounding any adjacent upstream element.

In some embodiments, the consumable may comprise an aerosol-cooling element which is adapted to cool the aerosol generated from the aerosol-forming substrate (by heat exchange) before being inhaled by the user.

The consumable may comprise a spacer element that defines a space or cavity between the aerosol-forming substrate and the downstream end of the consumable. The spacer element may comprise a cardboard tube. The spacer element may be circumscribed by the (paper) wrapping layer.

According to a third aspect of the present invention, there is provided a method of using the system according to the second aspect, the method comprising inserting the consumable into the device; and heating the article using the heater of the device.

In some embodiments the method may comprise inserting the article into a cavity within a body of the device and penetrating the article with the heating element of the device upon insertion of the article.

In a fourth aspect, the present invention provides a method of operating a smoking substitute device, the device for receiving a heated tobacco consumable, the method comprising: receiving a first user input to define one or more parameters of a first haptic feedback output; and outputting the first haptic feedback output at or proximal the conclusion of the consumable smoking cycle.

In some embodiments, the method comprises receiving a first user input (e.g. via input means on a UI as described above for the first aspect) to define the amplitude and/or the frequency and/or the duration and/or the pulse pattern/sequence of the first haptic feedback output.

The device may comprise a controller (as described above for the first aspect) and the method may comprise storing the parameter(s) defined in the first user input in a memory within the controller.

In some embodiments, the method comprises determining the conclusion of the consumable smoking cycle by monitoring the time elapsed since the commencement of the consumable smoking cycle and/or the number of puffs taken on the consumable by the user and/or the flow rate/duration of draw in each puff. The number and/or flow rate/duration of puffs may be detected using a puff sensor (as described above for the first aspect).

The method may comprise emitting a 'spent' signal from the controller at or proximal the end of the consumable smoking cycle to generate the first haptic feedback output having the parameter(s) defined by the first user input. The method may comprise generating the first haptic feedback output using a haptic element in a feedback means on the device (as described above for the first aspect).

In some embodiments, the method comprises monitoring the time elapsed between consecutive puffs on the consumable by the user (e.g. using the puff sensor) and outputting a second haptic feedback if the user has not puffed on the consumable for a predetermined length of time (e.g. greater than 30 seconds or 1 minute or greater) during the consumable smoking cycle.

The method may comprise emitting an 'idle' signal from the controller if the elapsed time exceeds the predetermined time to generate the second haptic feedback output. The method may comprise generating the second haptic feedback output using the haptic element in the feedback means on the device.

In some embodiments, the method comprises receiving a second user input (e.g. at the user input means) to define one or more parameters of the second haptic feedback output; and outputting the second haptic feedback output having the defined parameter(s) when the length of time between consecutive puffs by the user on the consumable exceeds the predetermined time.

In preferred embodiments, the method comprises selecting at least one different parameter in the second user input so that the second haptic feedback output has at least one different parameter (e.g. amplitude, frequency, duration, pulse pattern/sequence) than the first haptic feedback output. The method may comprise storing the defined parameter(s) for the second haptic feedback in the memory of the controller.

In some embodiments, the device comprises a power source (as described above for the first aspect) or may be connectable to a power source (e.g. a power source separate to the device) and the method further comprises outputting a third haptic feedback output when the power source is depleted or is approaching depletion.

The method may comprise emitting a 'depleted' signal from the controller if the power supply is approaching depletion to generate the third haptic feedback output. The method may comprise generating the third haptic feedback output using the haptic element in the feedback means on the device.

In some embodiments, the method comprises receiving a third user input (e.g. at the user input means) to define one or more parameters of the third haptic feedback output; and outputting the third haptic feedback output having the defined parameter(s) when the power source is approaching depletion.

In preferred embodiments, the method comprises selecting at least one different parameter in the third user input so that the third haptic feedback output has at least one different parameter (e.g. amplitude, frequency, duration, pulse pattern/sequence) than the first and second haptic feedback outputs. The method may comprise storing the defined parameter(s) for the third haptic feedback in the memory of the controller.

In some embodiments, the method further comprises selecting at least one parameter (e.g. amplitude and/or frequency and/or duration and/or pulse pattern/sequence) of a first visual feedback output (e.g. during the first user input), storing the at least one parameter in the memory of the controller and outputting a first visual feedback output simultaneously with the first haptic feedback output.

In some embodiments, the method further comprises selecting at least one parameter (e.g. amplitude and/or frequency and/or duration and/or pulse pattern/sequence) of a second visual feedback output (e.g. during the second user input), storing the at least one parameter in the memory of the controller and outputting a second visual feedback output simultaneously with the second haptic feedback output.

In some embodiments, the method further comprises selecting at least one parameter (e.g. amplitude and/or frequency and/or duration and/or pulse pattern/sequence) of a third visual feedback output (e.g. during the third user input), storing the at least one parameter in the memory of the controller and outputting a third visual feedback output simultaneously with the third haptic feedback output.

In some embodiments, the method further comprises selecting at least one parameter (e.g. amplitude and/or frequency and/or duration and/or pulse pattern/sequence) of a first audio feedback output (e.g. during the first user input), storing the at least one parameter in the memory of the controller and outputting a first audio feedback output simultaneously with the first haptic feedback output.

In some embodiments, the method further comprises selecting at least one parameter (e.g. amplitude and/or frequency and/or duration and/or pulse pattern/sequence) of a second audio feedback output (e.g. during the second user input), storing the at least one parameter in the memory of the controller and outputting a second audio feedback output simultaneously with the second haptic feedback output.

In some embodiments, the method further comprises selecting at least one parameter (e.g. amplitude and/or frequency and/or duration and/or pulse pattern/sequence) of a third audio feedback output (e.g. during the third user input), storing the at least one parameter in the memory of the controller and outputting a third audio feedback output simultaneously with the third haptic feedback output.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### SUMMARY OF THE FIGURES

So that the invention may be understood, and so that further aspects and features thereof may be appreciated, embodiments illustrating the principles of the invention will now be discussed in further detail with reference to the accompanying figures, in which:
Figure 1A is a schematic of a smoking substitute system;
Figure 1B is a schematic of a variation of the smoking substitute system of Figure 1A;
Figure 2A is a front view of a first embodiment of a smoking substitute system with the consumable engaged with the device;
Figure 2B is a front view of the first embodiment of the smoking substitute system with the consumable disengaged from the device;
Figure 2C is a section view of the consumable of the first embodiment of the smoking substitute system;
Figure 2D is a detailed view of an end of the device of the first embodiment of the smoking substitute system;
Figure 2E is a section view of the first embodiment of the substitute smoking system; and
Figure 3 is a flowchart illustrating method of operating a smoking substitute device for receiving a consumable in accordance with few embodiments of the present invention.

### DETAILED DESCRIPTION OF THE INVENTION

Aspects and embodiments of the present invention will now be discussed with reference to the accompanying figures. Further aspects and embodiments will be apparent to those skilled in the art. All documents mentioned in this text are incorporated herein by reference.

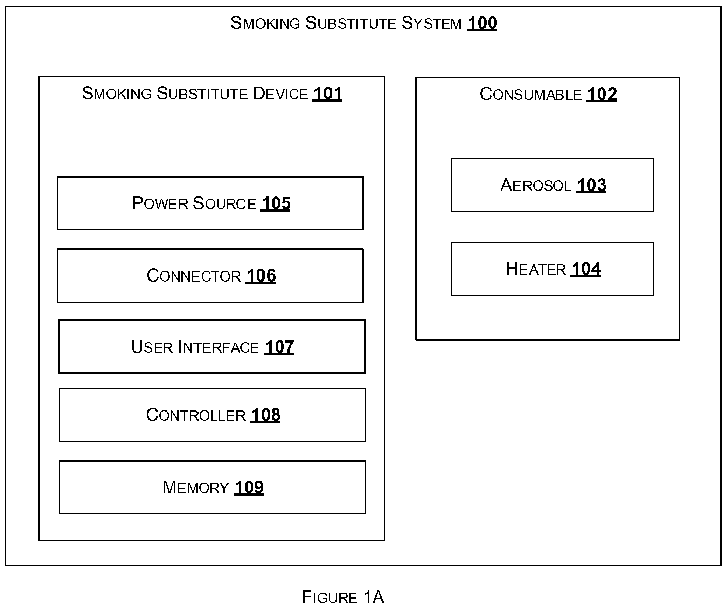

Figure 1A is a schematic providing a general overview of a smoking substitute system 100. The system 100 includes a substitute smoking device 101 and a consumable 102, which comprises an aerosol former 103. The system is configured to vaporise the aerosol former by heating the aerosol former 103 (so as to form a vapour/aerosol for inhalation by a user).

In the illustrated system, the heater 104 forms part of the consumable 102 and is configured to heat the aerosol former 103. Heat from the heater 104 vaporises the aerosol former 103 to produce a vapour. The vapour subsequently condenses to form an aerosol, which is ultimately inhaled by the user.

The system 100 further comprises a power source 105 that forms part of the device 101. In other embodiments the power source 105 may be external to (but connectable to) the device 101. The power source 105 is electrically connectable to the heater 104 such that the power source 105 is able to supply power to the heater 104 (i.e. for the purpose of heating the aerosol former 103). Thus, control of the electrical connection of the power source 105 to the heater 104 provides control of the state of the heater 104. The power source 105 may be a power store, for example a battery or rechargeable battery (e.g. a lithium ion battery).

The system 100 further comprises an I/O module comprising a connector 106 (e.g. in the form of a USB port, Micro USB port, USB-C port, etc.). The connector 106 is configured for connection to an external source of electrical power, e.g. a mains electrical supply outlet. The connector 106 may be used in substitution for the power source 105. That is the connector 106 may be electrically connectable to the heater 104 so as to supply electricity to the heater 104. In such embodiments, the device may not include a power source, and the power source of the system may instead comprise the connector 106 and an external source of electrical power (to which the connector 106 provides electrical connection).

In some embodiments, the connector 106 may be used to charge and recharge the power source 105 where the power source 105 includes a rechargeable battery.

The system 100 also comprises a user interface (Ul) 107. Although not shown, the UI 107 may include input means to receive commands (e.g. the first, second and third user input) from a user. The input means of the UI 107 allows the user to control at least one aspect of the operation of the system 100. The input means may, for example, be in the form of a button, touchscreen, switch, microphone, etc.

The UI 107 also comprises feedback means to convey information to the user. The feedback means may, for example, comprise a visual element (e.g. lights (e.g. LEDs), a display screen), an audio element (e.g. speaker), and a haptic element (e.g. vibration generator), etc.

The system 100 further comprises a controller 108 that is configured to control at least one function of the device 101. In the illustrated embodiment, the controller 108 is a component of the device 101, but in other embodiments may be separate from (but connectable to) the device 101. The controller 108 is configured to receive a first user input to define one or more parameters of a first haptic feedback output to indicate conclusion (or approach of the conclusion) of a consumable smoking cycle and generate a 'spent' signal to cause the haptic element in the feedback means to generate the haptic feedback output (having the parameter(s) defined in the first user input) at the conclusion of the consumable smoking cycle. The memory 109 stores the user-defined parameters (from the first/second/third user input).

The controller 108 is also configured to control the operation of the heater 104 and, for example, may be configured to control the voltage applied from the power source 105 to the heater 104. The controller 108 may be configured to toggle the supply of power to the heater 104 between an on state, in which the full output voltage of the power source 105 is applied to the heater 104, and an off state, in which the no voltage is applied to the heater 104.

Although not shown, the system 100 may also comprise a voltage regulator to regulate the output voltage from the power source 105 to form a regulated voltage. The regulated voltage may then be applied to the heater 104.

In addition to being connected to the heater 104, the controller 108 is operatively connected to the UI 107. Thus, the controller 108 may receive the first/second/third user input signal from the input means of the UI 107. Similarly, the controller 108 may transmit output signals (e.g. 'spent', 'idle' and 'depleted' output signals) to the UI 107. In response, the feedback means of the UI 107 may generate the first/second/third haptic/visual/audio feedback output.

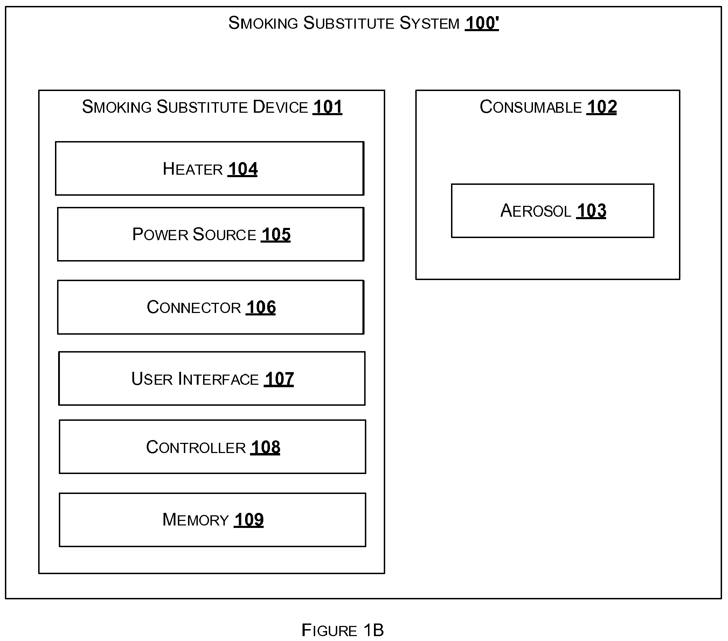

Figure 1B is a schematic showing a variation of the system 100 of Figure 1A. In the system 100' of Figure 1B, the heater 104 forms part of the device 101, rather than the consumable 102. In this variation, the heater 104 is electrically connected to the power source 105.

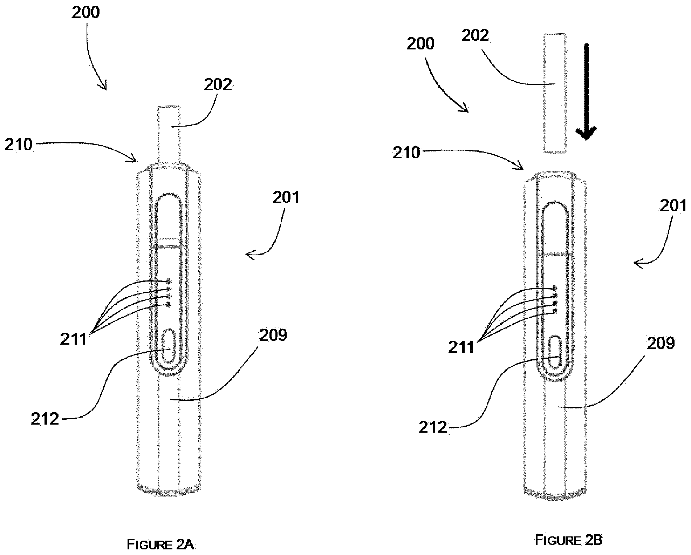

Figures 2A and 2B illustrate a heated-tobacco (HT) smoking substitute system 200. The system 200 is an example of the systems 100, 100' described in relation to Figures 1A or 1B. System 200 includes an HT device 201 and an HT consumable 202. The description of Figures 1A and 1B above is applicable to the system 200 of Figures 2A and 2B, and will thus not be repeated.

The device 201 and the consumable 202 are configured such that the consumable 202 can be engaged with the device 201. Figure 2A shows the device 201 and the consumable 202 in an engaged state, whilst Figure 2B shows the device 201 and the consumable 202 in a disengaged state.

The device 201 comprises a body 209 and cap 210. In use the cap 210 is engaged at an end of the body 209. Although not apparent from the figures, the cap 210 is moveable relative to the body 209. In particular, the cap 210 is slideable and can slide along a longitudinal axis of the body 209.

The device 201 comprises a feedback means (forming part of the UI of the device 201) having a visual element in the form of a plurality of light-emitting diodes (LEDs) 211 arranged linearly along the longitudinal axis of the device 201 and on an outer surface of the body 209 of the device 201. The feedback means also includes a haptic element (vibrator) and an audio element (speaker) which are not visible in Figures 2A and 2B. A button 212 is also arranged on an outer surface of the body 209 of the device 201 and is axially spaced (i.e. along the longitudinal axis) from the plurality of LEDs 211.

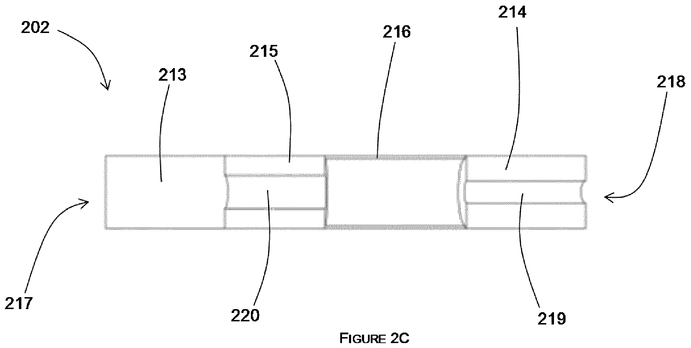

Figure 2C show a detailed section view of the consumable of 202 of the system 200. The consumable 202 generally resembles a cigarette. In that respect, the consumable 202 has a generally cylindrical form with a diameter of 7 mm and an axial length of 70 mm. The consumable 202 comprises an aerosol forming substrate 213, a terminal filter element 215, an upstream filter element 215 and a spacer element 216. In other embodiments, the consumable may further comprise a cooling element. A cooling element may exchange heat with vapour that is formed by the aerosol-forming substrate 213 in order to cool the vapour so as to facilitate condensation of the vapour.

The aerosol-forming substrate 213 is substantially cylindrical and is located at an upstream end 217 of the consumable 202, and comprises the aerosol former of the system 200. In that respect, the aerosol forming substrate 213 is configured to be heated by the device 201 to release a vapour. The released vapour is subsequently entrained in an airflow flowing through the aerosol-forming substrate 213. The airflow is produced by the action of the user drawing on a downstream 218 (i.e. terminal or mouth end) of the consumable 202.

In the present embodiment, the aerosol forming substrate 213 comprises tobacco material that may, for example, include any suitable parts of the tobacco plant (e.g. leaves, stems, roots, bark, seeds and flowers). The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon). For example, the aerosol-forming substrate 213 may comprise a gathered sheet of homogenised (e.g. paper/slurry recon) tobacco or gathered shreds/strips formed from such a sheet.

In order to generate an aerosol, the aerosol forming substrate 213 comprises at least one volatile compound that is intended to be vaporised/aerosolised and that may provide the user with a recreational and/or medicinal effect when inhaled. The aerosol-forming substrate 213 may further comprise one or more additives. For example, such additives may be in the form of humectants (e.g. propylene glycol and/or vegetable glycerine), flavourants, fillers, aqueous/non-aqueous solvents and/or binders.

The terminal filter element 214 is also substantially cylindrical, and is located downstream of the aerosol forming substrate 213 at the downstream end 218 of the consumable 202. The terminal filter element 214 is in the form of a hollow bore filter element having a bore 219 (e.g. for airflow) formed therethrough. The diameter of the bore 219 is 2 mm. The terminal filter element 214 is formed of a porous (e.g. monoacetate) filter material. As set forth above, the downstream end 218 of the consumable 202 (i.e. where the terminal filter 214 is located) forms a mouthpiece portion of the consumable 202 upon which the user draws. Airflow is drawn from the upstream end 217, thorough the components of the consumable 202, and out of the downstream end 218. The airflow is driven by the user drawing on the downstream end 218 (i.e. the mouthpiece portion) of the consumable 202.

The upstream filter element 215 is located axially adjacent to the aerosol-forming substrate 213, between the aerosol-forming substrate 213 and the terminal filter element 214. Like the terminal filter 214, the upstream filter element 215 is in the form of a hollow bore filter element, such that it has a bore 220 extending axially therethrough. In this way, the upstream filter 215 may act as an airflow restrictor. The upstream filter element 215 is formed of a porous (e.g. monoacetate) filter material. The bore 220 of the upstream filter element 214 has a larger diameter (3 mm) than the terminal filter element 214.

The spacer 216 is in the form of a cardboard tube, which defines a cavity or chamber between the upstream filter element 215 and the terminal filter element 214. The spacer 216 acts to allow both cooling and mixing of the vapour/aerosol from the aerosol-forming substrate 213. The spacer has an external diameter of 7 mm and an axial length of 14mm.

Although not apparent from the figure, the aerosol-forming substrate 213, upstream filter 215 and spacer 216 are circumscribed by a paper wrapping layer. The terminal filter 214 is circumscribed by a tipping layer that also circumscribes a portion of the paper wrapping layer (so as to connect the terminal filter 214 to the remaining components of the consumable 202). The upstream filter 215 and terminal filter 214 are circumscribed by further wrapping layers in the form of plug wraps.

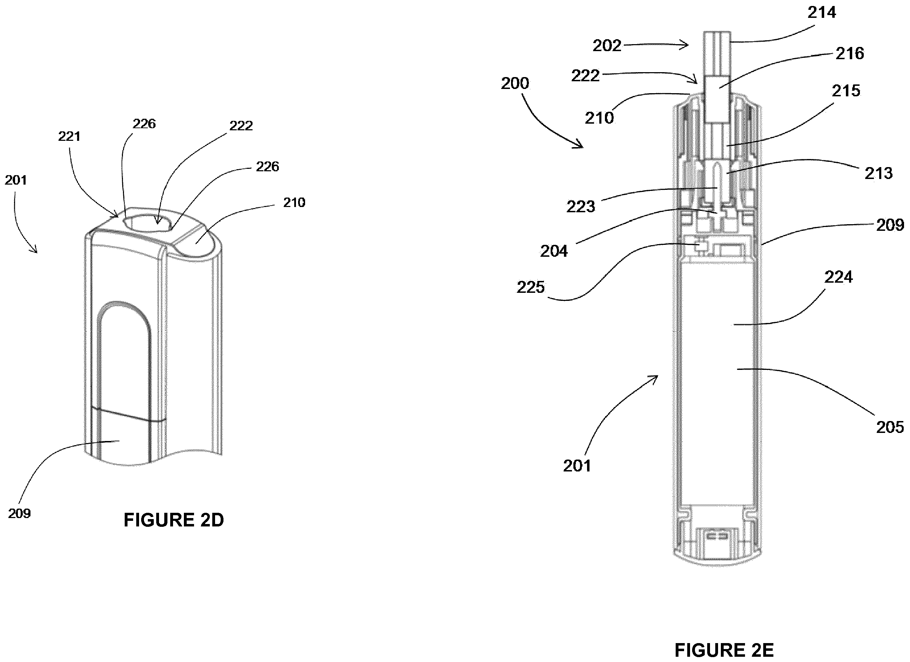

Returning now to the device 201, Figure 2D illustrates a detailed view of the end of the device 201 that is configured to engage with the consumable 202. The cap 210 of the device 201 includes an opening 221 to an internal cavity 222 (more apparent from Figure 2D) defined by the cap 210. The opening 221 and the cavity 222 are formed so as to receive at least a portion of the consumable 202. During engagement of the consumable 202 with the device 201, a portion of the consumable 202 is received through the opening 221 and into the cavity 222. After engagement (see Figure 2B), the downstream end 218 of the consumable 202 protrudes from the opening 221 and thus protrudes also from the device 201. The opening 221 includes laterally disposed notches 226. When a consumable 202 is received in the opening 221, these notches 226 remain open and could, for example, be used for retaining a cover to cover the end of the device 201.

Figure 2E shows a cross section through a central longitudinal plane through the device 201. The device 201 is shown with the consumable 202 engaged therewith.

The device 201 comprises a heater 204 comprising heating element 223. The heater 204 forms part of the body 209 of the device 201 and is rigidly mounted to the body 209. In the illustrated embodiment, the heater 204 is a rod heater with a heating element 223 having a circular transverse profile. In other embodiments the heater may be in the form of a blade heater (e.g. heating element with a rectangular transverse profile) or a tube heater (e.g. heating element with a tubular form).

The heating element 223 of the heater 204 projects from an internal base of the cavity 222 along a longitudinal axis towards the opening 221. As is apparent from the figure, the length (i.e. along the longitudinal axis) of the heating element is less than a depth of the cavity 222. In this way, the heating element 223 does not protrude from or extend beyond the opening 221.

When the consumable 202 is received in the cavity 222 (as is shown in Figure 2E), the heating element 223 penetrates the aerosol-forming substrate 213 of the consumable 202. In particular, the heating element 223 extends for nearly the entire axial length of the aerosol-forming substrate 213 when inserted therein. Thus, when the heater 204 is activated, heat is transferred radially from an outer circumferential surface the heating element 223 to the aerosol-forming substrate 213.

The device 201 further comprises an electronics cavity 224. A power source, in the form of a rechargeable battery 205 (a lithium ion battery), is located in electronics cavity 224.

The device 201 includes a connector (i.e. forming part of an IO module of the device 201) in the form of a USB port 206. The connector may alternatively be, for example, a micro-USB port or a USB-C port for examples. The USB port 206 may be used to recharge the rechargeable battery 205.

The device 201 includes the controller 208 (not shown) located in the electronics cavity 224. The controller comprises a microcontroller mounted on a printed circuit board (PCB). The USB port 206 is also connected to the controller 208 (i.e. connected to the PCB and microcontroller).

The controller 208 is configured to notify the user that the consumable smoking cycle is nearing completion or completed via a first haptic feedback output to the user. The first haptic feedback output is a haptic feedback sequence/pattern previously defined by the user in the first user input (and stored in the memory 109 of the device. The first haptic feedback output may also be configured with a user-defined amplitude, frequency, duration or number of haptic vibrations.

In one example, the controller 208 receives a first user input, via the input means, to define the parameters of the first haptic feedback output that indicate the conclusion of the consumable smoking routine. The controller 208 generates a 'spent' output signal at the conclusion of the consumable smoking cycle which is received at the feedback means to cause the haptic element to generate the first haptic feedback output (having the defined parameters).. The controller 208 determines the duration of the consumable smoking cycle based on usage pattern of the device 201 by the user taking into account the time elapsed since the commencement of the cycle, the number of puffs taken by the user (using a puff sensor 225) and the length/flow rate of each puff.

By providing feedback about the conclusion of the consumable smoking routine, the users are continuously updated on when the experience was nearly over, thereby providing an intuitive user experience.

The puff sensor 225 is configured to detect a user drawing (i.e. inhaling) at the downstream end 218 of the consumable 202. The puff sensor 225 may, for example, be in the form of a pressure sensor, flowmeter or a microphone. The puff sensor 225 is operatively connected to the controller 208 in the electronics cavity 224, such that a signal from the puff sensor 225, indicative of a puff state (i.e. drawing or not drawing) sends a puff signal to the controller 208 (and can thus be responded to by the controller 208).

The controller 208, further receives a second user input, via the input means, to define one of more parameters of a second haptic feedback output. At least one parameter of the second haptic feedback output is different from the parameters in the first haptic feedback output.

The controller 208 generates an 'idle' signal output if no puff signal from the puff sensor is detected for a predetermined length of time (say 1 minute). The 'idle' output signal is received by the feedback means and causes the haptic element to output the second haptic feedback output.

Furthermore, the controller 208 also receives a third user input, via the input means, to define one or more parameters of a third haptic feedback output to notify the user about the depletion of the battery or indication to the user that the device 201 needs to be recharged. In one example, the controller 208 determines the current battery level of the device 201 and alert the user about the depletion of the current battery level to the user so that the user is aware of when the device 201 is to be recharged. At least one parameter of the third haptic feedback output is different from the parameters in the first and second haptic feedback outputs.

The controller is also configured to control the LEDs 211 in response to (e.g. a detected) a condition of the device 201 or the consumable 202. For example, the controller may control the LEDs to indicate whether the device 201 is in an on state or an off state (e.g. one or more of the LEDs may be illuminated by the controller when the device is in an on state).

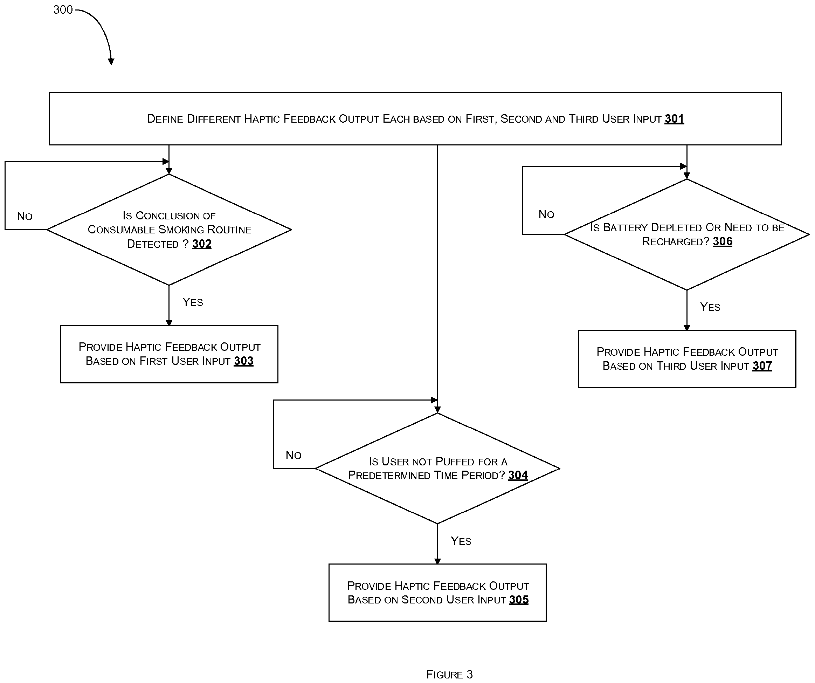

Figure 3 illustrates flowchart of method of operating the device.

As illustrated in Figure 3, the method 300 includes one or more blocks implemented by the controller 208 of the device 201. The method 300 may be described in the general context of controller executable instructions. Generally, controller executable instructions can include routines, programs, objects, components, data structures, procedures, modules, and functions, which perform particular functions or implement particular abstract data types.

The order in which the method 300 is described is not intended to be construed as a limitation, and any number of the described method blocks can be combined in any order to implement the method 300. Additionally, individual blocks may be deleted from the method 300 without departing from the scope of the subject matter described herein. Furthermore, the method 300 can be implemented in any suitable hardware, software, firmware, or combination thereof.

At block 301, the controller 208 receives a first user input, a second user input and a third user input from the user via the input means of the device 201. The first, second and third user input is received to define a first, second and third haptic feedback output respectively for various functions or operations of the device. The first, second and third haptic feedback output may also be defined with different amplitude, frequency, duration or number of haptic vibrations.

In one example, the controller 208 receives a first user input, via the input means, to define the first haptic feedback output that indicates the conclusion of the consumable smoking cycle.

The controller 208, further receives a second user input, via the input means, to define the second haptic feedback output to notify the user about non-puffing of the user for a predetermined time period during the consumable smoking routine. The parameters defined in the first user input are different from the parameters defined in the second user input. Furthermore, the controller 208 also receives a third user input to define the third haptic feedback output to notify the user about the depletion of the battery or indication to the user that the device 201 needs to be recharged. In one example, the parameters defined in the third user input are different from the parameters defined in the first and the second user inputs.

At block 302, the controller 208 detects as to whether the conclusion of the consumable smoking routine is reached. If the controller 208 detects the conclusion of the consumable smoking routine, then the method proceeds to block 303 along the "YES" path to generate the 'spent' output signal to cause the haptic element in the feedback means to generate the user-defined first haptic feedback. Otherwise, the method proceeds to block 302 along the "NO" path to continuously detect the conclusion of the consumable smoking routine.

At block 304, the controller 208 detects whether the user has not puffed for a predetermined time period (determined from the time elapsed between puff signals generated by the puff sensor). The controller 208 receives the idle time period when the device 201 is not being used by the user and detects that the user has not puffed for the predetermined time period when the idle time period exceeded the predetermined time period. If the controller 208 detects that the user has not puffed for the predetermined time period, then the method proceeds to block 305 along the "YES" path to generate the 'idle' output signal to cause the haptic element of the feedback means to generate the user-defined second haptic feedback output.

Otherwise, the method proceeds to block 304 along the "NO" path to continuously detect the non-usage of the device 201.

At block 306, the controller 208 detects the depletion of the battery or indication to the user that the device 201 needs to be recharged. In one example, the controller 208 determines the current battery level of the device 201 and detects the depletion of the current battery level or indication that the device 201 needs to be recharged based on the battery level. Based on the detection of depletion of battery level or indication of recharge required, the method proceeds to block 307 along the "YES" path to generate the 'depleted' output signal to cause the haptic element of the feedback means to generate the user-defined third haptic feedback output. Otherwise, the method proceeds to block 306 along the "NO" path to continuously detect the depletion of the current battery level or indication that the device 201 needs to be recharged.

In addition to the haptic feedback, the controller 208 may also enable visual or audio feedback to the user (using the visual/audio elements in the feedback means). By allowing the user to the select different, user-defined haptic feedback parameters for different situations (e.g. cycle end, battery depletion, idle time), the user is provided with an intuitive, versatile feedback mechanism that can be used even in loud or bright environments.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute device for receiving a heated tobacco consumable, the device configured to receive a first user input to define one or more parameters of a first haptic feedback output; and output the first haptic feedback output at or proximal the conclusion of the consumable smoking cycle.

2. A device according to claim 1 wherein the first user input is to define the amplitude and/or the frequency and/or the duration and/or the pulse pattern/sequence of the first haptic feedback output

3. A device according to claim 1 or 2 comprising user input means for receiving the first user input and configured to generate a first user input signal upon receipt of the first user input.

4. A device according to claim 3 comprising a controller for receiving the first user input signal from the user input means, the controller comprising a memory for storing the first haptic feedback output parameter(s) defined in the first user input.

5. A device according to any one of the preceding claims comprising a puff sensor for generating a puff signal upon detecting a user puff on the consumable, the device further configured to output a second haptic feedback output if the puff sensor has not generated a puff signal for a predetermined length of time during the consumable smoking cycle.

6. A device according to claim 5 configured to receive a second user input to define one or more parameters of the second haptic feedback output; and output the second haptic feedback output having the user-defined parameter(s) when puff sensor has not generated a puff signal for a predetermined length of time during the consumable smoking cycle.

7. A device according to any one of the preceding claims comprising a power source, the device being further configured to output a third haptic feedback output when the power source is depleted or is approaching depletion.

8. A device according to claim 7 configured to receive a third user input to define one or more parameters of the third haptic feedback output; and output the third haptic feedback output having the user-defined parameter(s) when the power supply is approaching depletion.

9. A method of operating a smoking substitute device, the device for receiving a heated tobacco consumable, the method comprising: receiving a first user input to define one or more parameters of a first haptic feedback output; and outputting the first haptic feedback output at or proximal the conclusion of the consumable smoking cycle.

10. A method according to claim 9 comprising receiving a first user input to define the amplitude and/or the frequency and/or the duration and/or the pulse pattern/sequence of the first haptic feedback output.

11. A method according to claim 9 or 10 comprising receiving a second user input to define one or more parameters of a second haptic feedback output; and outputting the second haptic feedback output having the defined parameter(s) when the length of time between consecutive puffs by the user on the consumable exceeds a predetermined time

12. A method according to any one of claims 9 to 11 comprising receiving a third user input to define one or more parameters of a third haptic feedback output; and outputting the third haptic feedback output having the defined parameter(s) when a power source is approaching depletion.

13. A method according to any one of claims 9 to 12 further comprising selecting at least one parameter of a first visual and/or audio feedback output and outputting a first visual and/or audio feedback output simultaneously with the first haptic feedback output.

14. A method according to claim 11 further comprising selecting at least one parameter of a second visual and/or audio feedback output and outputting a second visual and/or audio feedback output simultaneously with the second haptic feedback output.

15. A method according to claim 12 further comprising selecting at least one parameter of a third visual and/or audio feedback output and outputting a third visual and/or audio feedback output simultaneously with the third haptic feedback output.
